# EUROPÄISCHE PATENTSCHRIFT

(11) **EP 2 621 662 B1**
(45) Veröffentlichungstag und Bekanntmachung des Hinweises auf die Patenterteilung: **01.05.2019**
(21) Anmeldenummer: 10763131.9
(22) Anmeldetag: 30.09.2010
(51) Int. Cl.: B23K 26/04, B23K 26/06

(54) **VORRICHTUNG UND VERFAHREN FÜR DIE BEARBEITUNG VON MATERIAL MIT FOKUSSIERTER ELEKTROMAGNETISCHER STRAHLUNG**
DEVICE AND METHOD FOR PROCESSING MATERIAL BY MEANS OF FOCUSED ELECTROMAGNETIC RADIATION
DISPOSITIF ET PROCÉDÉ POUR LE TRAITEMENT D'UNE MATIÈRE AU MOYEN D'UN RAYONNEMENT ÉLECTROMAGNÉTIQUE FOCALISÉ

(43) Veröffentlichungstag der Anmeldung: 07.08.2013
(73) Patentinhaber: WaveLight GmbH, 91058 Erlangen (DE)
(72) Erfinder: WARM, Berndt, 90571 Schwaig (DE); RIEDEL, Peter, 90489 Nuernberg (DE); GORSCHBOTH, Claudia, 90411 Nuernberg (DE); WOITTENNEK, Franziska, 01129 Dresden (DE)
(74) Vertreter: von Hellfeld, Axel
(86) Internationale Anmeldenummer: PCT/EP2010/005976
(87) Internationale Veröffentlichungsnummer: WO 2012/041351

(56) Entgegenhaltungen:
- WO-A1-2007/096136
- DE-A1-102007 060 344
- DE-A1-102008 015 403

## Beschreibung

Die Erfindung betrifft eine Vorrichtung und ein Verfahren für die Bearbeitung von Material mit fokussierter elektromagnetischer Strahlung.

Bei den hier in Rede stehenden Vorrichtungen handelt es sich insbesondere um optische Systeme, die von zum Beispiel Lasern oder LEDs als Strahlungsquellen erzeugte elektromagnetische Strahlung auf oder in ein zu bearbeitendes Material lenken, formen und fokussieren. Als Materialbearbeitung kommt hier zum Beispiel eine Materialstrukturierung im Mikrobereich in Betracht, wie Sie zum Beispiel in Halbleitern durchgeführt wird oder auch in metallischen Materialien. Insbesondere kann die Erfindung Anwendung finden bei ophthalmologischen optischen Systemen, insbesondere in der refraktiven Hornhautchirurgie, wie zum Beispiel LASIK.

Bei der Materialbearbeitung mit fokussierter elektromagnetischer Strahlung kommt es in der Regel auf eine genaue Positionierung des Fokus an, insbesondere in Richtung der elektromagnetischen Strahlung (üblicherweise mit "z-Richtung" bezeichnet). Die Position des Fokus wird üblicherweise auch mit "Fokuslage" bezeichnet, wobei dieser Begriff nicht nur den vorstehend erläuterten Ort des Fokus in Richtung der Strahlung (die sog. Fokustiefe) erfasst, sondern allgemeiner auch die Position und Orientierung der fokussierten Strahlung, also zum Beispiel einen Versatz der Strahlung in Bezug auf die optische Achse des Systems oder eine Winkelstellung dazu.

In der US 2002/0171028 ist eine Vorrichtung zur Fokuskontrolle beschrieben. Dabei wird reflektiertes Licht durch einen Abbildungsstrahlengang zur Interferenz mit einem zweiten Strahlenbündel gebracht und es wird eine interferometrische Messung und Steuerung durchgeführt.

Auch bei der US 6,666,857 B2 wird eine Fokussteuerung mittels einer interferometrischen Wellenfrontkontrolle durchgeführt. Die aktive Wellenfrontsteuerung während der Photoablation am menschlichen Auge wird durch eine Kombination von adaptiven Spiegeln erreicht.

In der US 2004/0021851 wird eine optische Anordnung bestehend aus einem Laser und nachfolgender Strahlformungsoptik zur Vermessung der Brennweite einer unbekannten Linse verwendet. Die Messung der Brennweite wird dabei durch das Fokussieren auf eine Referenzoberfläche mit verschiedenen Abständen durchgeführt. Es wird der rückreflektierte Anteil der Strahlung detektiert. Die Spotdurchmesser werden dann mit den jeweiligen Abständen ausgewertet. Mittels der "Newtonschen" Relation Z Z'=f² wird die Brennweite bestimmt. Ein optisches Gitter, welches nicht näher beschrieben ist, wird zur Auskopplung des rückreflektierten Anteils der Strahlung verwendet. Der Jones-Matrix Formalismus wird ebenfalls zur Berechnung der Brennweite herangezogen. Die Genauigkeit des Verfahrens liegt bei 1%.

Die WO 2007/096136 A1 beschreibt eine Vorrichtung zum Detektieren der Fokuslage eines optischen Systems mit einer teilreflektierenden Fläche am zu vermessenden Fokus, einer Kamera zur Aufnahme eines von der genannten Fläche reflektierten Bildes, und mit einem Rechner zum Auswerten des von der Kamera aufgenommenen Bildes. Dabei ist im Strahlengang des optischen Systems vor dem fokussierenden Abbildungssystem ein optisches Element angeordnet, welches das genannte Bild in Abhängigkeit von der Fokuslage beeinflusst. Eine Steuerung der Fokuslage erfolgt über Elemente der fokussierenden Optik. Dieses Dokument offenbart die Merkmale des Oberbegriffs der Ansprüche 1 und 11.

Nachfolgend soll die vorliegende Erfindung insbesondere mit Blick auf die sog. fs-LASIK (Femtosekundenlasik) beschrieben und erläutert werden, wobei sich der Einsatz der Erfindung bei anderen Materialbearbeitungen, bei denen eine genaue Steuerung der Fokuslage gewünscht ist, analog ergibt.

Der Erfindung liegt die Aufgabe zugrunde, bei einer Bearbeitung von Material mit fokussierter elektromagnetischer Strahlung in einfacher und zuverlässiger Weise eine Steuerung, insbesondere eine Regelung, der Fokuslage zu ermöglichen.

Hierzu stellt die Erfindung bereit eine Vorrichtung für die Bearbeitung von Material mit fokussierter elektromagnetischer Strahlung, aufweisend:
- eine Quelle, die elektromagnetische Strahlung emittiert,
- Mittel zum Richten der Strahlung auf das Material,
- Mittel zum Fokussieren der Strahlung auf oder in dem Material,
- eine Einrichtung zum Erzeugen eines Musters in dem Strahlengang der elektromagnetischen Strahlung,
- eine zumindest teilreflektierende Fläche im Strahlengang vor dem Fokus der fokussierten Strahlung, wobei das genannte Muster über zumindest einen Teil der genannten Mittel zum Richten und der genannten Mittel zum Fokussieren auf die zumindest teilreflektierende Fläche abgebildet wird,
- zumindest einen Detektor, auf den ein Bild des Musters von der genannten Fläche reflektiert wird und der dem Bild entsprechende elektrische Signale erzeugt, wobei das Bild Information über die Lage des Fokus enthält,
- einen Rechner, der die genannten elektrischen Signale empfängt und der programmiert ist, das genannte Bild zu verarbeiten, um ein von der Fokuslage abhängiges elektrisches Signal zu erzeugen, und
- ein Divergenzstellelement, das in dem genannten Strahlengang angeordnet und eingerichtet ist, das genannte elektrische Signal des Rechners zu empfangen, um eine Divergenz der elektromagnetischen Strahlung in Abhängigkeit von dem Signal zu ändern, Der Rechner ist erfindungsgemäß eingerichtet, Änderungen der Bildgröße oder von Punktäbstanden des genannten Bildes als Funktion von Divergenzänderungen der Strahlung zu ermitteln und daraus, das von der Fokuslage abhängige elektrische Signal zu erzeugen.

Mit einer solchen Vorrichtung ist es möglich, über das Divergenzstellelement die Fokuslage zu steuern oder zu regeln indem der Rechner bei der Bildverarbeitung die Information über die Fokuslage ableitet und dann, wenn die Ist-Fokuslage nicht einer gewünschten Soll-Fokuslage entspricht, ein Signal zu erzeugen, gemäß dem das Divergenzstellelement die Strahldivergenz so ändert, dass die Ist-Fokuslage der Soll-Fokuslage entspricht. Durch Änderung der Strahldivergenz ändert sich die Fokuslage, ohne dass dabei die Fokussierungsmittel (also die fokussierende Optik im engeren Sinne) notwendig betätigt werden müssten. Wird die Strahldivergenz vergrößert, wandert der Fokus in Strahlrichtung und wird die Strahldivergenz verkleinert, wandert der Fokus entgegen der Strahlrichtung.

Gemäß einer bevorzugten Ausgestaltung der Erfindung ist die genannte teilreflektierende Fläche an einem Ort der Vorrichtung angeordnet, an dem auch die elektromagnetische Strahlung die Vorrichtung in Richtung auf das zu bearbeitende Material verlässt.

Eine andere bevorzugte Ausgestaltung der Erfindung sieht vor, dass es sich bei dem zu bearbeitenden Material um Augengewebe, insbesondere die Hornhaut, handelt. Bei dieser Anwendung der Erfindung wird mit der Vorrichtung zum Beispiel das sog. "Flap" mit insbesondere einem Femtosekundenlaser erzeugt. Beim Schneiden der Hornhaut zur Erzeugung des Flaps bei der fs-LASIK ist der Schnitt durch Steuerung der Fokuslage besonders präzise und möglichst eben, d.h. fokustiefentreu zu führen. Dabei wird typischerweise eine Glasplatte mit einer sog. Applanationsfläche gegen die Hornhaut gedrückt, um das Auge zu fixieren und eine Referenzfläche für das Schneiden des Flaps im Stroma der Hornhaut zu erhalten. Die fokussierten Pulse des Lasers schneiden dann in einer Tiefe von typischerweise etwa 100 µm relativ zur Applanationsfläche einen ebenen Schnitt in die Hornhaut. Am Rande des Schnitts wird die Schnitttiefe verringert, so dass der Rand des Flaps bis auf ein "Scharnier" gelöst werden kann, um das Flap zur Seite zu klappen.

Die oben beschriebene Erfindung ermöglicht eine exakte und gleichbleibende Einstellung der Schnitttiefe und überwindet damit im Stand der Technik bisweilen auftretende Probleme, die durch unerwünschte Variationen der Schnitttiefe aufgrund von sich während der Prozedur ändernden Fokuslagen ergeben können. Mit der Erfindung ist es möglich, Variationen in der Schnitttiefe auf wenige µm zu reduzieren.

Bei heute eingesetzten typischen Systemen für die fs-LASIK wird die Schnitttiefe in Bezug auf die sog. Applanationsfläche, also die Fläche, mit der die genannte Glasplatte die Hornhaut in eine definierte Ebene drückt, in der Regel vor der Behandlung eingestellt, indem Schnitte an Probekörpern durchgeführt werden.

Der vorliegenden Erfindung liegt die Erkenntnis zugrunde, dass trotz einer derartigen Bestimmung der Schnitttiefe mit Probekörpern gleichwohl unerwünschte Variationen der voreingestellten Schnitttiefen auftreten können. Sowohl in der Zeitspanne zwischen der Festlegung der Schnitttiefe mit Probekörpern als auch während der Operation selbst (also der Erzeugung des Schnitts) kann es zu unerwünschten Änderungen der Schnitttiefe kommen, und zwar insbesondere durch
- eine Änderung der Divergenz des Laserstrahls, insbesondere durch thermische Änderungen der Laserkomponenten oder anderer optischer Komponenten, und auch durch eine Drift der Laserstrahlrichtung,
- Veränderungen der optischen Komponenten für die Fokussierung, wiederum insbesondere durch thermische Änderungen, und
- Fertigungsungenauigkeiten der Flächen der Glasplatte, die zur Erzeugung der Applanationsfläche an das Auge gedrückt wird.

Im Stand der Technik werden nach der einmal erfolgten Voreinstellung der Schnitttiefe mit zum Beispiel dem genannten Probekörper, später auftretende Änderungen in den Komponenten, die Einfluss auf die Fokuslage haben, nicht mehr erkannt und insbesondere auch nicht mehr korrigiert. Die Erfindung überwindet diesen Mangel und ermöglicht wahlweise:
- die Erkennung von Änderungen der Laserstrahldivergenz, insbesondere aufgrund thermischer Effekte;
- die Erkennung von Änderungen der Fokussierungseigenschaften der Fokussierungsoptik, insbesondere aufgrund thermischer Effekte;
- die Erkennung von Änderungen in der Form des Laserstrahls;
- die Erkennung von Änderungen in der Strahlrichtung;
- die Erkennung von Fertigungsungenauigkeiten hinsichtlich der genannten Applanationsfläche und deren Vermessung; und
- die Überprüfung des Strahlwegs bis unmittelbar vor die Materialbearbeitung, also insbesondere die Schnittführung bei der fs-LASIK; und weiterhin
- die Erkennung von Systemfehlern im Laser oder im Strahlengang, insbesondere während der Operation.

Weitere bevorzugte Ausgestaltungen der Erfindung sind in den abhängigen Ansprüchen beschrieben. Die Erfindung stellt weiter ein Verfahren nach Anspruch 11 bereit.

Nachfolgend werden Ausführungsbeispiele der Erfindung anhand der Zeichnung näher erläutert. Es zeigt:
- Fig. 1: schematisch eine Vorrichtung für die Bearbeitung von Material mit fokussierter elektromagnetischer Strahlung;
- Fig. 2: ein Ausführungsbeispiel für eine Maske im Strahlengang der elektromagnetischen Strahlung; und

- Fig. 3: schematisch den Einfluss von Änderungen der Strahldivergenz auf ein Muster.

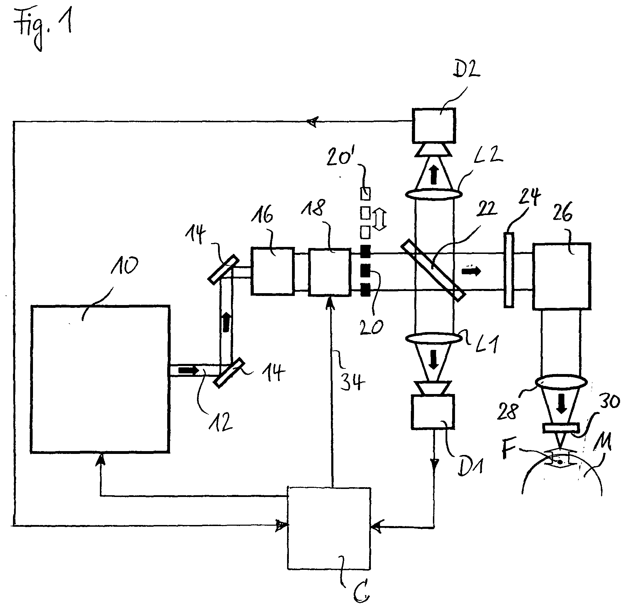

Die in Fig. 1 dargestellte Vorrichtung für die Bearbeitung von Material mit fokussierter elektromagnetischer Strahlung betrifft das Schneiden eines sog. Flaps ("Deckelchens") in der Femtosekunden-LASIK, also die Erzeugung eines Schnitts in der Kornea eines Auges, welches hier das Material M darstellt.

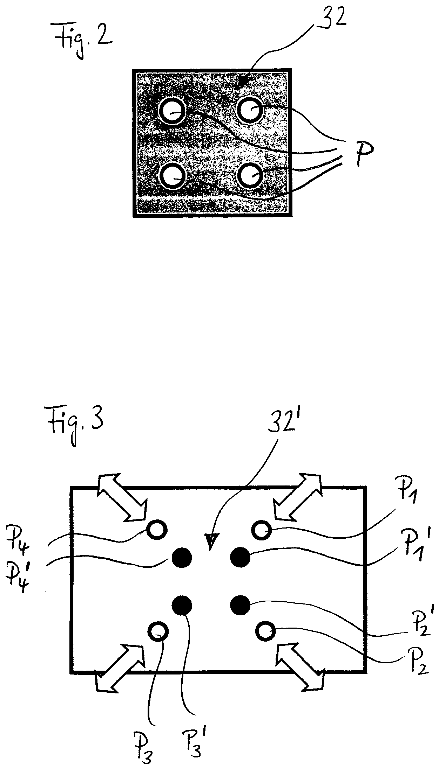

Als Strahlungsquelle 10 dient ein Femtosekundenlaser der für diese Anwendung bekannten Art. Die vom Femtosekundenlaser emittierter Strahlung 12 wird über zwei Umlenkspiegel 14 einem Strahlaufweiter 16 zugeführt, der den Strahldurchmesser aufweitet. Der aufgeweitete Laserstrahl wird in ein Divergenzstellelement 18 gerichtet, also eine Einrichtung, mit der die Divergenz des Laserstrahls vergrößert oder verkleinert werden kann. Als Divergenzstellelement kommt insbesondere in Betracht ein Teleskop mit verstellbaren Linsen, ein System deformierbarer Spiegel oder deformierbare Linsen. Danach passiert der Laserstrahl einen Mustererzeuger 20, zum Beispiel eine Lochmaske der weiter unten näher beschriebenen Art. Der Musterezeuger 20 erzeugt eine inhomogene Verteilung der Intensität des Strahls über dessen Querschnitt. Fig. 2 zeigt beispielhaft eine Lochmaske mit der ein Muster erzeugt wird, das vier Punkte in den Ecken eines Quadrats aufweist. Auch andere Strukturen als die gezeigten Löcher sind für die Mustererzeugung geeignet. Durch Änderung der Divergenz des Laserstrahls mittels des Divergenzstellelements 18 ändern sich die Abmessungen der vom Strahl hinter der Maske erzeugten Bilder der Maske, also der Muster. Der Mustererzeuger ist vorzugsweise in den Strahlengang einschwenkbar und aus ihm herausschwenkbar.

Nach Passieren der Maske trifft der aufgeweitete und in seiner Divergenz eingestellte Laserstrahl auf einen Strahlteiler 22. Ein Teil des Strahls wird vom Strahlteiler 22 in Fig. 1 nach unten über eine Linse L1 auf einen ersten Detektor D1 abgelenkt. Ein anderer Teil des Laserstrahls passiert den Strahlteiler 22 geradlinig. Dieser Teilstrahl trifft auf einen Shutter 24 und bei offenem Shutter 24 auf eine Umlenkeinheit 26, mit der der Strahl geführt und in Bezug auf das Auge M positioniert wird. Eine Fokussieroptik ist schematisch durch eine Linse 28 dargestellt. Eine Glasplatte ist mit einer (unteren) Applanationsfläche 30 gegen die Hornhaut des Auges M gedrückt, wie es bei der fs-LASIK zum Schneiden des Flaps üblich ist. Der Fokus F des Laserstrahls wird über die Fokussierungsoptik (Linse 28) eingestellt und liegt beim Schneiden des Flaps in einer im Wesentlichen zur Strahlrichtung senkrechten Ebene in der Hornhaut in zum Beispiel 100 µm Tiefe zur Hornhautoberfläche.

Wird mit dem Divergenzstellelement 18 die Divergenz des Laserstrahls verändert, so ändert sich auch bei ansonsten gleichbleibenden Einstellungen der optischen Komponenten die Lage des Fokus F was in Fig. 1 durch den Doppelpfeil symbolisiert ist.

Die Applanationsfläche 30 der Glasplatte ist teilreflektierend, so dass die Abbildung des mit dem Mustererzeugers 20 erzeugten Musters auf der Applanationsfläche 30 zurückreflektiert wird und über den Strahlteiler 22 und eine Linse L2 zu einem zweiten Detektor D2 gelangt. Die reflektierende Applanationsfläche liegt so nahe am Fokus F wie es das zu bearbeitende Material erlaubt.

Die beiden Detektoren D1, D2 sind hochauflösende elektronische Kameras und erzeugen aus den von ihnen aufgenommenen Abbildungen des mit dem Mustererzeuger 20 erzeugten Musters elektrische Signale, die für eine Bildverarbeitung an einen Rechner C übergeben werden.

Aufgrund der Funktion des Strahlteilers 22 empfängt der erste Detektor D1 Bilder des Musters, die von der Strahlungsquelle 10 zum ersten Detektor D1 gelangen, ohne an der teilreflektierenden Fläche 30 reflektiert worden zu sein. Der zweite Detektor D2 empfängt Bilder des Musters, die von der teilreflektierenden Fläche 30 zurückreflektiert werden. Die Linsen L1 bzw. L2 formatieren die Bilder auf den Detektoren für eine maximale Auflösung.

Aufgrund der beschriebenen Anordnung der optischen Komponenten in Bezug auf die Detektoren D1 und D2 enthalten die von den Detektoren aufgenommenen Bilder des Musters Informationen über den optischen Zustand sämtlicher optischer Komponenten der Vorrichtung, insbesondere Informationen über die Divergenz des Strahls bei Durchtritt durch den Mustererzeuger 20. Der erste Detektor D1 erfasst diese Informationen für den Strahlungsgang vor dem Strahlteiler 22 und der zweite Detektor D2 erfasst diese Informationen bezüglich aller Komponenten im Strahlungsgang von der Quelle 10 bis zur Applanationsfläche 30.

Der Mustererzeuger 20 kann zum Beispiel als in den Strahlengang einschiebbare Platte mit Löchern (Lochmaske) ausgebildet sein. In Fig. 1 ist die Verschiebbarkeit der Lochmaske mit dem Doppelpfeil angedeutet und die Position, in der die Lochmaske aus dem Strahlengang entfernt ist mit dem Bezugszeichen 20'.

Anstelle der Lochmaske können auch elektrooptische Mittel vorgesehen sein, die im Strahlengang verbleiben und so ansteuerbar sind, dass wahlweise ein freier Durchgang des Strahls möglich ist oder eine Maske im Strahlengang entsteht, mit der ein Muster erzeugt wird.

Die Funktion der Vorrichtung gemäß Fig. 1 zur Steuerung oder Regelung der Lage des Fokus F ist wie folgt:
Wie eingangs erläutert ist, kann es bei Vorrichtungen der in Rede stehenden Art zu unerwünschten Verschiebungen der Fokuslage kommen aufgrund von insbesondere durch thermische Effekte bedingte Änderungen der optischen Eigenschaften von Komponenten im Strahlengang der von der Quelle 10 emittierten Strahlung. Eine Änderung der Fokuslage tritt insbesondere dann auf, wenn sich die Strahldivergenz ändert. Ändert sich die Divergenz des Laserstrahls, werden die Bilder der Maske (also die Bilder des von der Maske erzeugten Musters) auf den Detektoren D1, D2 größer oder kleiner, je nach dem, ob die Divergenz größer oder kleiner wird. Dabei sind Eigenschaften des Bildes, insbesondere die Größe des auf der Applanationsfläche 30 erzeugten Bildes der Maske auch ein Maß für die Fokuslage, d.h. das auf der Applanationsfläche erzeugte Abbild des vom Mustererzeuger 20 erzeugten Strahlungsmusters enthält Informationen über die Lage des Fokus F. Dies kann die Bildverarbeitung im Rechner C nutzen, um Steuersignale für das Divergenzstellelement 18 zu errechnen, mit denen die Divergenz des Strahls so gesteuert wird, dass der Fokus F eine gewünschte Lage hat.

Fig. 2 zeigt ein Beispiel für eine Lochmaske mit vier Punkten P. Fig. 3 zeigt wie auf der Applanationsfläche 30 die Abbildung dieser Lochmaske sich in Abhängigkeit von variierenden Divergenzen der Strahlung ändern kann. Diese Änderungen sind zum Beispiel durch die Doppelpfeile in Fig. 3 markiert. So können zum Beispiel bei einer symmetrischen Änderung der Divergenz die usprünglichen Positionen der Punkte P1, P2, P3, P4 nach Innen in die entsprechenden Positionen P1', P2', P3' bzw. P4' wandern, was bedeutet, dass der Fokus F näher an die Applanationsfläche 30 heranrückt. Wenn die gewünschte Fokuslage aber der Ausgangsposition der Punkte P1, P2, P3, und P4 entspricht, kann durch Änderung der Divergenz (zum Beispiel Vergrößerung der Divergenz abhängig von der Art der optischen Komponenten) diese Ausgangslage wiederhergestellt werden. Dies kann in der Art eines Regelkreises durchgeführt werden, so dass in der Zeit zwischen der Vorbereitung einer Operation, zum Beispiel einer optischen Kalibrierung des Systems bis zum Ende der Operation immer die gewünschte Fokustiefe mit großer Genauigkeit beibehalten wird.

Aus dem vorstehend Gesagten, ergibt sich, dass das Bild der Maske auf der Fläche 30 eine Information enthält über die Lage des Fokus F. Weiterhin ergibt sich, dass durch Einstellung der Divergenz mittels des Divergenzstellelements 18 das Bild des Musters auf der Fläche änderbar ist.

Mit diesen funktionalen Abhängigkeiten ist es möglich, die optische Vorrichtung gemäß Fig. 1 bezüglich der Fokuslage zu kalibrieren, d.h. zum Beispiel experimentell in den zu erwartenden Schwankungsbereichen mit einem Testauge oder dergleichen jeder Fokuslage ein Bild auf der Fläche 30 zuzuordnen. Dieses Bild wird dann vom Detektor D2 aufgenommen und im Rechner C zusammen mit die Fokuslage charakterisierenden Daten, wie der Fokustiefe, abgespeichert. Für die Bildverarbeitung können im Rechner C Daten abgelegt werden, die experimentell (empirisch) dadurch gewonnen werden, dass jeweilig Muster-Bildern bestimmte Fokuslagen zugeordnet werden. Diese funktionale Zuordnung kann zum Beispiel in Tabellenform erfolgen oder auch durch eine empirisch gewonnene mathematische Funktion. Für das gegebene optische System der Vorrichtung sind Änderungen der Bildgrößen des Musters eine eindeutige Funktion von Divergenzänderungen der Strahlung und weiterhin sind Änderungen der Fokuslage ebenfalls eine eindeutige Funktion der Änderung der Bildgrößen und damit eine eindeutige Funktion der Divergenzänderungen. Diese funktionalen Abhängigkeiten können für ein gegebenes optisches System, also eine Vorrichtung gemäß Fig. 1, für die gegebenen Komponenten vorab empirisch ermittelt und im Rechner C in der beschriebenen Weise abgelegt werden.

In dem Regelkreis fungiert das Divergenzstellelement 18 als Stellelement. Die Störgröße des Regelkreises wird aus den Bildern der Detektoren bestimmt. Zum Beispiel können aus dem Bild der Maske Längeninformationen (Abmessungen) gewonnen werden, zum Beispiel Abmessungen zwischen den Punkten gemäß Fig. 3. Durch Interpolation können die der Analyse zugrundegelegten Größen genauer sein als die Pixelabmessungen der Detektoren D1, D2.

Zum Beispiel kann gemäß Fig. 3 der Abstand zwischen den gefüllten Punkten und den leeren Punkten durch Bildverarbeitung im Rechner C ermittelt werden und eine empirisch gewonnen Funktion, welche die Änderung der Abstände in Abhängigkeit von einer Änderung der Fokuslage beinhaltet, kann herangezogen werden, um durch Änderung der Divergenz mittels des Divergenzstellelements 18 die eine aus den gespeicherten Funktionen berechnete Lage der Punkte und damit die gewünschte Fokuslage wiederherzustellen. Dies geschieht alles relativ in Bezug auf eine anfänglich experimentell (empirisch) ermittelte Referenzsituation, in der die Fokuslage mit zum Beispiel einem Testauge oder dergleichen gemessen wird und diese Fokuslage zum Beispiel genau die gewünschten Schnitttiefe beim Schneiden eines Flaps in der fs-LASIK ist. Zu dieser gewünschten Referenztiefe gehören dann ganz bestimmte Abmessungen der Punkte im Musterbild gemäß den Fig. 2 und 3 und der Rechner C steuert das Divergenzstellelement 18 so, dass vor und während der Operation die der gewünschten Fokuslage entsprechenden Punktabstände beibehalten werden.

Störgrößen sind dabei insbesondere thermische Änderungen der optischen Weglänge auf dem Weg der Strahlung von der Quelle 10 bis zum Strahlteiler 22, Divergenzänderungen auf diesem Weg oder auch andere Änderungen der optischen Komponenten. Dies führt zu Änderungen der Bilder auf beiden Detektoren D1, D2, wobei die Änderung auf dem Detektor D1 bereits alle Informationen über diese Störgrößen enthält.

Auf dem Weg vom Strahlteiler 22 zur Applanationsfläche 30 auftretende Änderungen der optischen Komponente, insbesondere thermisch bedingte Änderungen, oder auch Fehlpositionierungen der Applanationsfläche 30 führen zu Änderungen des Bildes auf dem Detektor D2, ohne dass dies die vom Detektor D1 aufgenommenen Bilder beeinflusst.

Damit lässt sich mit dem Rechner auch eine Auswertung der Störgrößen vornehmen und die Störungen können gegebenenfalls lokalisiert werden, d.h. bestimmten optischen Komponenten zugeordnet werden.

Das beschriebene System ermöglicht über die beschriebene Regelung der Fokuslage hinaus noch weitere wesentliche Überwachungen des Systems mit Blick auf eine erhöhte Operationssicherheit. So kann zum Beispiel mit dem Rechner eine Überwachungsfunktion implementiert werden, um Strahlunterbrechungen, Änderungen der Strahlelliptizität, Verschmutzungen, Defekte der Optik etc. festzustellen. All dies wirkt sich auf das Bild des Musters, wie es auf einem oder beiden Detektoren D1, D2 erscheint, aus und kann ausgewertet werden, um Gefährdungen des Patienten auszuschließen, zum Beispiel durch Abbruch der Operation bei Auftreten von Abweichungen der vorstehend genannten Parameter von vorgegebenen Sollwerten. Um den Rechenaufwand bei der Bildverarbeitung im Rechner C möglichst gering zu halten, empfiehlt es sich, das vom Mustererzeuger 20 erzeugte Muster möglichst einfach zu wählen. Mit der in Fig. 2 gezeigten relativ einfachen Maske wird dies erreicht. Mit der dargestellten Punktanordnung kann durch Summation von Spalten und Zeilen jeweils eine eindimensionale Verteilung mit jeweils zwei Spitzen erzeugt werden. Der Abstand dieser Spitzen kann ohne besonders viel Rechenaufwand mit Ortsauflösungen besser als ein Pixelabstand (durch Interpolation) bestimmt werden.

Lochmasken können zum Beispiel verwirklicht werden durch Bohrungen in Metallplatten, geschwärzte Filme, holographische Elemente, Linsenarrays etc. Einfache Masken können mit Bildverarbeitung ohne große Rechenaufwand und in quasi Echtzeit verarbeitet werden, während komplexere Masken mehr Kontrollfunktionen und Fehleranalysen ermöglichen.

In Abwandlung des vorstehend mit Blick auf Fig. 1 beschriebenen Ausführungsbeispiels ist es möglich, den Detektor D1 mit der zugehörigen Linse L1 wegzulassen oder durch einen Spiegel zu ersetzen. Bei Einsatz eines Spiegels anstelle des Detektors D1 kann eine Strahlumlenkung hinzugefügt werden, die den Strahl entweder zum Fokus F durchlässt oder zum Spiegel blockiert.

Eine andere Abwandlung des Ausführungsbeispiels gemäß Fig. 1 kann vorsehen, den Detektor D2 und die zugehörige Linse L2 wegzulassen oder durch einen Spiegel zu ersetzen. Ist dabei der Shutter 24 geschlossen, ermöglicht dies die Messung der Strahlverteilung des austretenden Strahls. Ist der Shutter 24 offen, dann wird die auf dem Detektor D1 schon vorhandene Bildverteilung durch eine zweite Bildverteilung (von der Fläche 30 reflektiert) überlagert. Beide Bilder können relativ zueinander verarbeitet werden, zum Beispiel kann das eine Bild vom anderen abgezogen werden. Dabei können die beiden Bilder räumlich voneinander getrennt werden, was die Bildverarbeitung vereinfacht.

Werden der Detektor D1 und die zugehörige Linse L1 weggelassen, dann kann der Shutter 24 durch einen klappbaren Spiegel ersetzt werden, um je nach Klappzustand des Spiegels zeitlich versetzt die hier interessierenden Bilder mit nur einem Detektor D2 (Kamera) aufzunehmen.

Es ist auch möglich, statt zweidimensionaler Kameras als Detektoren hierfür Zeilenkameras einzusetzen, wenn für den Mustererzeuger Linienmuster vorgegeben werden.

Auch ist es möglich, die Fokuslage hinsichtlich Position und Winkel durch eine Gestaltung der Umlenkspiegel 14 zu steuern. Auch die Fokusposition, also der Ort des Fokus in einer Ebene senkrecht zur Strahlrichtung, wirkt sich auf das Bild des Musters auf der Applanationsfläche 30 aus und kann durch Bildverarbeitung im Rechner C für die Steuerung der Fokusposition mittels der Umlenkspiegel, die als motorisch verstellbare Elemente vom Rechner C steuerbar ausgeführt werden, herangezogen werden. Hierzu kann der Rechner C analog wie oben anhand der Fokustiefe im Zusammenhang mit der Divergenz ausgeführt ist, auch empirisch (experimentell) so programmiert werden, dass er die Umlenkspiegel 14 so steuert, dass die Fokusposition senkrecht zur Strahlrichtung auf einen gewünschten Wert, wie er vom Rechner vorgegeben wird, eingeregelt wird. Dabei wird diese Soll-Position des Fokus beim Schneiden eines Flaps in der durch die konstante Fokustiefe definierten Ebene beibehalten.

Entsprechendes gilt auch für die Richtung der Strahlung am Ort des Fokus F, die ebenfalls einen Niederschlag im Bild des Musters auf der Fläche 30 findet und somit einer Steuerung und Regelung analog den obigen Ausführungen zum Zusammenhang zwischen Fokustiefe und Divergenz unterworfen werden kann durch Steuerung der Umlenkspiegel 14 mittels des Computers C.

### Bezugszeichenliste

- 10: Strahlungsquelle
- 12: Strahlung
- 14: Umlenkspiegel
- 16: Strahlaufweiter
- 18: Divergenzstellelement
- 20: Mustererzeuger
- 22: Strahlteiler
- 24: Shutter
- 26: Strahlführung
- 28: Fokussierung
- 30: Applanationsfläche
- F: Fokus
- L1: Linse
- L2: Linse
- D1: Detektor
- D2: Detektor
- C: Computer
- 32: Muster
- P: Punkt
- 34: Signal

## Patentansprüche

1. Vorrichtung für die Bearbeitung von Material (M) mit fokussierter elektromagnetischer Strahlung, aufweisend:
- eine Quelle (10), die elektromagnetische Strahlung (12) emittiert,
- Mittel (14, 16, 18, 22, 26) zum Richten der Strahlung auf das Material (M),
- Mittel (28) zum Fokussieren der Strahlung auf oder in dem Material (M),
- eine Einrichtung (20) zum Erzeugen eines Musters (32) in dem Strahlengang der elektromagnetischen Strahlung,
- eine teilreflektierende Fläche (30) im Strahlengang vor dem Fokus (F) der fokussierten Strahlung, wobei das genannte Muster (32) über zumindest einen Teil der genannten Mittel zum Richten und der genannten Mittel zum Fokussieren auf der genannten teilreflektierenden Fläche (30) abgebildet wird,
- zumindest einen Detektor (D1, D2) auf den ein Bild des Musters (32) von der genannten Fläche (30) reflektiert wird und der dem genannten Bild entsprechende elektrische Signale erzeugt, wobei das Bild eine Information über die Lage des Fokus (F) enthält,
- einen Rechner (C), der die genannten elektrischen Signale empfängt und der programmiert ist, das genannte Bild zu verarbeiten, um ein von der Fokuslage abhängiges elektrisches Signal (34) zu erzeugen, und
- ein Divergenzstellelement (18), das in dem genannten Strahlengang angeordnet ist,
**dadurch gekennzeichnet, dass**
- der Rechner (C) eingerichtet ist, Änderungen der Bildgröße oder von Punktabständen des genannten Bildes als Funktion von Divergenzänderungen der Strahlung zu ermitteln und daraus, das von der Fokuslage abhängige elektrische Signal zu erzeugen und
- das Divergenzstellelement (18) eingerichtet ist, das genannte elektrische Signal (34) des Rechners (C) zu empfangen und die Divergenz der elektromagnetischen Strahlung in Abhängigkeit von dem Signal zu ändern.

2. Vorrichtung nach Anspruch 1 **dadurch gekennzeichnet, dass** die genannte Fläche (30) dort angeordnet ist, wo die elektromagnetische Strahlung die Vorrichtung verlässt.

3. Vorrichtung nach einem der Ansprüche 1 oder 2 **dadurch gekennzeichnet, dass** das Material (M) ein Augengewebe, insbesondere die Hornhaut, ist.

4. Vorrichtung nach einem der vorhergehenden Ansprüche, **dadurch gekennzeichnet, dass** die Quelle (10) ein Laser, insbesondere ein Femtosekundenlaser ist.

5. Vorrichtung nach einem der vorhergehenden Ansprüche, **dadurch gekennzeichnet, dass** die genannte Fläche (30) eine Applanationsfläche ist, die eingerichtet ist, an eine Hornhaut angedrückt zu werden.

6. Vorrichtung nach einem der vorhergehenden Ansprüche, **dadurch gekennzeichnet, dass** das Muster (32) zwei oder mehr Punkte (P) aufweist.

7. Vorrichtung nach einem der vorhergehenden Ansprüche, **dadurch gekennzeichnet, dass** das Divergenzstellelement (18) ein Teleskop, einen deformierbaren Spiegel, oder eine deformierbare Linse aufweist.

8. Vorrichtung nach einem vorhergehenden Ansprüche, **dadurch gekennzeichnet, dass** zwei Detektoren (D1, D2) vorgesehen sind, von denen einer (D1) von der Quelle (10) emittierte Strahlung detektiert, und der andere (D2) von der Fläche (30) reflektierte Strahlung.

9. Vorrichtung nach einem der vorhergehenden Ansprüche, **dadurch gekennzeichnet, dass** die das Muster (32) erzeugende Einrichtung (20) im Strahlengang der elektromagnetischen Strahlung hinter dem Divergenzstellelement (18) angeordnet ist.

10. Vorrichtung nach einem der vorhergehenden Ansprüche, **dadurch gekennzeichnet**, das Muster (32) matrixartig angeordnete Punkte (P) aufweist.

11. Verfahren für die Bearbeitung von Material (M) mit fokussierter elektromagnetischer Strahlung, folgende Schritte aufweisend:
- Erzeugen elektromagnetischer Strahlung (12) mittels einer Quelle (10),
- Richten der Strahlung auf das zu bearbeitende Material,
- Fokussieren der Strahlung auf oder in dem Material,
- Erzeugen eines Musters (32) mittels der elektromagnetischen Strahlung,
- Bereitstellen zumindest einer teilreflektierenden Fläche (30) im Strahlengang der elektromagnetischen Strahlung vor einem Fokus (F) der fokussierten Strahlung,
- Abbilden des genannten Musters auf der genannten zumindest teilreflektierenden Fläche (30),
- Detektieren von der genannten Fläche (30) reflektierter Strahlung mittels zumindest eines Detektors (D1, D2), wobei die detektierten Bilder eine Information über die Lage des Fokus (F) enthalten,
- Erzeugen von elektrischen Signalen, die die detektierten Bilder repräsentieren, und
- Verarbeiten der genannten elektrischen Signale in einem Rechner (C), um ein von der Fokuslage abhängiges elektrische Signal (34) zu erzeugen, **gekennzeichnet durch**
- Ermitteln von Änderungen der Bildgröße oder von Punktabständen der Bilder als Funktion von Divergenzänderungen der Strahlung mittels eines Rechners (C) und erzeugen von elektrischen Signalen, die von der Fokuslage abhängen, und
- Einstellen der Divergenz der Strahlung in Abhängigkeit von dem genannten elektrischen Signal (34).

## Claims

1. Apparatus for processing material (M) using focused electromagnetic radiation, having:
- a source (10) emitting electromagnetic radiation (12),
- means (14, 16, 18, 22, 26) for directing the radiation onto the material (M),
- means (28) for focusing the radiation on or in the material (M),
- a device (20) for producing a pattern (32) in the beam path of the electromagnetic radiation,
- a partially reflective surface (30) in the beam path upstream of the focus (F) of the focused radiation, wherein said pattern (32) is imaged on said partially reflective surface (30) via at least a part of said means for directing and said means for focusing,
- at least one detector (D1, D2), onto which an image of the pattern (32) is reflected by said surface (30) and which produces electrical signals that correspond to said image, wherein the image contains information relating to the position of the focus (F),
- a computer (C), which receives said electrical signals and is programmed to process said image in order to produce an electrical signal (34) that is dependent on the focus position, and
- a divergence actuating element (18) arranged in said beam path,
**characterized in that**
- the computer (C) is set up to ascertain changes in the image size or in point spacings of said image as a function of divergence changes of the radiation and to produce therefrom the electrical signal that is dependent on the focus position, and
- the divergence actuating element (18) is set up to receive said electrical signal (34) of the computer (C) and to change the divergence of the electromagnetic radiation in dependence on the signal.

2. Apparatus according to Claim 1, **characterized in that** said surface (30) is arranged where the electromagnetic radiation leaves the apparatus.

3. Apparatus according to one of Claims 1 or 2, **characterized in that** the material (M) is eye tissue, in particular the cornea.

4. Apparatus according to one of the preceding claims, **characterized in that** the source (10) is a laser, in particular a femtosecond laser.

5. Apparatus according to one of the preceding claims, **characterized in that** said surface (30) is an applanation surface, which is set up to be pressed against the cornea.

6. Apparatus according to one of the preceding claims, **characterized in that** the pattern (32) has two or more points (P).

7. Apparatus according to one of the preceding claims, **characterized in that** the divergence actuating element (18) has a telescope, a deformable mirror or a deformable lens.

8. Apparatus according to one of the preceding claims, **characterized in that** two detectors (D1, D2) are provided, one (D1) of which detects radiation emitted by the source (10) and the other (D2) of which detects radiation reflected by the surface (30) .

9. Apparatus according to one of the preceding claims, **characterized in that** the device (20) producing the pattern (32) is arranged in the beam path of the electromagnetic radiation downstream of the divergence actuating element (18).

10. Apparatus according to one of the preceding claims, **characterized in that** the pattern (32) has points (P) that are arranged in the manner of a matrix.

11. Method for processing material (M) using focused electromagnetic radiation, having the following steps:
- producing electromagnetic radiation (12) using a source (10),
- directing the radiation onto the material to be processed,
- focusing the radiation on or in the material,
- producing a pattern (32) using the electromagnetic radiation,
- providing at least one partially reflective surface (30) in the beam path of the electromagnetic radiation upstream of a focus (F) of the focused radiation,
- imaging said pattern onto said at least partially reflective surface (30),
- detecting radiation reflected by said surface (30) using at least one detector (D1, D2), wherein the detected images contain information relating to the position of the focus (F),
- producing electrical signals that represent the detected images, and
- processing said electrical signals in a computer (C) in order to produce an electrical signal (34) that is dependent on the focus position,
**characterized by**
- ascertaining changes in the image size or in point spacings of the images as a function of divergence changes of the radiation using a computer (C) and producing electrical signals that are dependent on the focus position, and
- setting the divergence of the radiation in dependence on said electrical signal (34).

## Revendications

1. Ensemble de traitement d'un matériau (M) par un rayonnement électromagnétique focalisé, l'ensemble présentant :
une source (10) qui émet le rayonnement électromagnétique (12),
des moyens (14, 16, 18, 22, 26) qui orientent le rayonnement sur le matériau (M),
des moyens (28) qui focalisent le rayonnement sur ou dans le matériau (M),
un dispositif (20) de formation d'un motif (32) dans le parcours du rayonnement électromagnétique,
une surface (30) partiellement réfléchissante prévue dans le parcours des rayons en avant du foyer (F) du rayonnement focalisé, une image dudit motif (32) étant formée sur ladite surface (30) partiellement réfléchissante par l'intermédiaire d'au moins une partie desdits moyens d'orientation et desdits moyens de focalisation,
au moins un détecteur (D1, D2) sur lequel une image du motif (32) de ladite surface (30) est réfléchie et qui forme des signaux électriques qui correspondent à ladite image, l'image contenant une information sur la position du foyer (F),
un calculateur (C) qui reçoit lesdits signaux électriques et qui est programmé pour traiter ladite image en vue de former un signal électrique (34) qui dépend de la position du foyer et
un élément (18) d'ajustement de la divergence disposé dans ledit parcours des rayons,
**caractérisé en ce que**
le calculateur (C) est conçu pour déterminer des modifications de la taille de l'image ou des distances entre les points de ladite image en fonction de modifications de la divergence du rayonnement et à partir de là, pour former le signal électrique dépendant de la position du foyer et
**en ce que** l'élément (18) d'ajustement de la divergence est conçu pour recevoir ledit signal électrique (34) du calculateur (C) et modifier la divergence du rayonnement électromagnétique en fonction du signal.

2. Ensemble selon la revendication 1, **caractérisé en ce que** ladite surface (30) est disposée là où le rayonnement électromagnétique quitte l'ensemble.

3. Ensemble selon l'une des revendications 1 ou 2, **caractérisé en ce que** le matériau (M) est un tissu oculaire et en particulier la peau de la cornée.

4. Ensemble selon l'une des revendications précédentes, **caractérisé en ce que** la source (10) est un laser et en particulier un laser à femtosecondes.

5. Ensemble selon l'une des revendications précédentes, **caractérisé en ce que** ladite surface (30) est une surface de planage conçue pour être imprimée sur la peau de la cornée.

6. Ensemble selon l'une des revendications précédentes, **caractérisé en ce que** le motif (32) présente deux ou plusieurs points (P).

7. Ensemble selon l'une des revendications précédentes, **caractérisé en ce que** l'élément (18) d'ajustement de la divergence présente un télescope, un miroir déformable ou une lentille déformable.

8. Ensemble selon l'une des revendications précédentes, **caractérisé en ce qu'**il présente deux détecteurs (D1, D2) parmi lesquels l'un (D1) détecte le rayonnement émis par la source (10) et l'autre (D2) le rayonnement réfléchi par la surface (30) .

9. Ensemble selon l'une des revendications précédentes, **caractérisé en ce que** le dispositif (20) qui forme le motif (32) est disposé dans le parcours du rayonnement électromagnétique en arrière de l'élément (18) d'ajustement de la divergence.

10. Ensemble selon l'une des revendications précédentes, **caractérisé en ce que** le motif (32) présente des points (P) disposés en matrice.

11. Procédé de traitement de matériau (M) par un rayonnement électromagnétique, le procédé présentant les étapes suivantes :
formation d'un rayonnement électromagnétique (12) au moyen d'une source (10),
orientation du rayonnement sur le matériau à traiter,
focalisation du rayonnement sur ou dans le matériau,
formation d'un motif (32) au moyen du rayonnement électromagnétique,
placement d'au moins une surface (30) partiellement réfléchissante dans le parcours du rayonnement électromagnétique en avant d'un foyer (F) du rayonnement focalisé,
formation de l'image dudit motif sur ladite surface (30) au moins partiellement réfléchissante,
détection du rayonnement réfléchi par ladite surface (30) au moyen d'au moins un détecteur (D1, D2), les images détectées contenant une information sur la position du foyer (F),
formation de signaux électriques qui représentent les images détectées,
traitement desdits signaux électriques dans un calculateur (C) en vue de former un signal électrique (34) qui dépend de la position du foyer,
**caractérisé par** les étapes qui consistent à :
déterminer au moyen d'un calculateur (C) des modifications de la taille de l'image ou des distances entre points des images en fonction de modifications de la divergence du rayonnement et former des signaux électriques qui dépendent de la position du foyer et
ajuster la divergence du rayonnement en fonction dudit signal électrique (34).
